# EUROPEAN PATENT SPECIFICATION

(11) **EP 3 451 933 B1**
(45) Date of publication and mention of the grant of the patent: **15.12.2021**
(21) Application number: 17793276.1
(22) Date of filing: 03.05.2017
(51) Int. Cl.: A61B 8/00, A61B 8/14, A61B 8/08, A61N 7/00

(54) **SYSTEMS AND COMPUTER READABLE MEDIA FOR ISCHEMIC INJURY PROTECTIVE ULTRASOUND**
SYSTEME UND COMPUTERLESBARE MEDIEN FÜR SCHÜTZENDEN ULTRASCHALL VON ISCHÄMISCHEN LÄSIONEN
SYSTÈMES ET SUPPORTS LISIBLES PAR ORDINATEUR DESTINÉS À DES ULTRASONS PROTECTEURS DE LÉSION ISCHÉMIQUE

(30) Priority: 03.05.2016 US 201662331104 P
(43) Date of publication of application: 13.03.2019
(73) Proprietor: University Of Virginia Patent Foundation, Charlottesville, VA 22903 (US)
(72) Inventor: OKUSA, Mark D., Charlottesville, VA 22901 (US); GIGLIOTTI, Joseph C., Ruckersville, VA 22968 (US); HOSSACK, John A., Charlottesville, VA 22901 (US)
(74) Representative: Isarpatent
(86) International application number: PCT/US2017/030893
(87) International publication number: WO 2017/192754

(56) References cited:
- US-A- 5 647 367
- US-A1- 2004 071 664
- US-A1- 2008 097 206
- US-A1- 2008 183 077
- US-A1- 2010 204 576
- US-A1- 2014 058 292
- US-A1- 2014 058 292
- US-A1- 2014 194 726
- US-A1- 2015 025 372
- JOSEPH C. GIGLIOTTI ET AL: "Ultrasound Modulates the Splenic Neuroimmune Axis in Attenuating AKI", JOURNAL OF THE AMERICAN SOCIETY OF NEPHROLOGY., vol. 26, no. 10, 2 February 2015 (2015-02-02), pages 2470-2481, XP055574454, US ISSN: 1046-6673, DOI: 10.1681/ASN.2014080769

## Description

### TECHNICAL FIELD

The presently disclosed subject matter relates to computer programs, systems, and computer readable media for therapeutic use of ultrasound. The computer programs, systems, and computer readable media for therapeutic ultrasound provide for the protection of tissues from ischemia-reperfusion injury.

### BACKGROUND

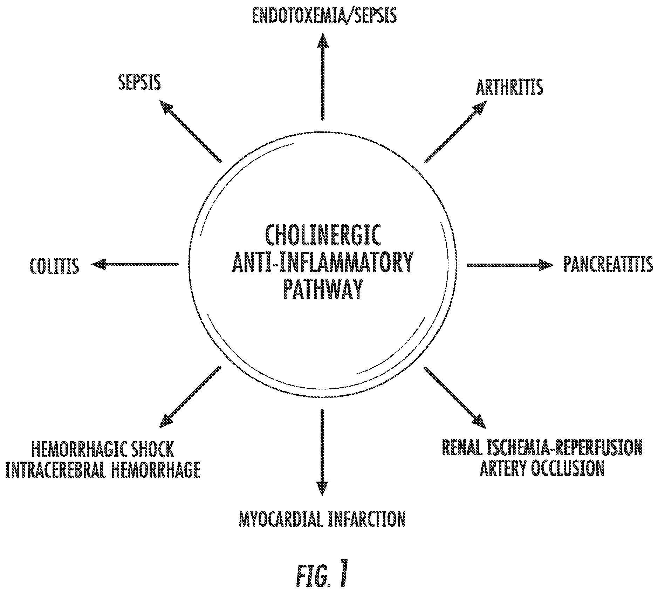

Acute Kidney Injury (AKI) is a significant clinical problem. Acute kidney injury resulting from hypotension, ischemia-reperfusion (IR), nephrotoxins such as radiocontrast agents, and sepsis to name a few causes, is an abrupt cessation of kidney function leading to fluid overload, electrolyte acid-base imbalance and inability to rid the body of metabolic toxins; in severe cases it leads to death (Figure 1). Therefore, prevention and treatment of AKI is an important clinical problem, as mortality in patients with AKI, especially in critically ill patients, remains alarmingly high (40-60% of ICU patients) despite substantial advances in techniques of resuscitation and renal replacement therapy. Over a 15-yr period (1988-2002) the incidence of AKI continued to increase; there were 1,083,745 discharges with AKI. The greatest predisposing factor to the development of AKI is chronic kidney disease (CKD). There are 20-40 million people in the USA with CKD and they have a high risk factor for sustaining AKI. Currently there are no FDA approved drugs for AKI. Small sample size, poorly timed administration during trials, and adverse effects associated with drugs have hampered the success of clinical trials.

The pathogenesis is complex. Following ischemia-reperfusion injury (IRI) there are alterations in renal medullary vascular tone. Medullary vascular congestion due to leukostasis contributes to a decrease in blood flow. The cascade of events following IR leading to endothelial cell dysfunction and activation of tissue-resident and infiltrating includes the coordinated action of cytokines/chemokines, reactive oxygen intermediates and adhesion molecules. Modulation of central and peripheral neural activity to target organ function has gained considerable interest recently. Current techniques for modulating neural activity include pharmacological agents or mechanical devices. Surgical and invasive procedures are required for most pharmacological agents that require direct application and for mechanical devices. Thus, additional approaches for treating AKI, and other related diseases or disorders with similarly complex pathogenesis, represent a long-felt and ongoing need in the art. For example, ischemic-reperfusion injuries can occur in other organ systems (such as the heart), and methods described in the context of AKI may be used in these other applications.

Joseph C. Gigliotti et al. disclose in "Ultrasound Modulates the Splenic Neuroimmune Axis in Attenuating AKI" an ultrasound application by an ultrasound transducer of an ultrasound machine. First a kidney is localized in real time using conventional B-mode imaging with a frequency of 14 MHz and an on-screen imaging mechanical index of 0.99. Cadence imaging begins with a frequency of 7 MHz and an imaging mechanical index of 0.16. The ultrasound application consists of ultrasound pulses administered with a bursting mechanical index of 1.2. The ultrasound pulses are 1 second in duration and applied once every 6 seconds for 2 minutes.

US 2014/0058292 A1 discloses a system for use in managing a neuromodulation therapy including an ultrasound transducer array controlled by a control unit to deliver ultrasound waveforms for causing modulation of neural tissue in a patient.

US 2008/0097206 A1 discloses contrast agent augmented ultrasound thrombus treatment, where clots are cleared by acoustic thrombolysis (sonothrombolysis) using ultrasound and contrast agents (microbubbles).

### SUMMARY

The present invention provides a system for effecting ultrasound-based protection from ischemia/reperfusion injury (IRI) and/or mitigating in a subject an inflammatory condition, disease or disorder according to independent claim 1 as well as a corresponding non-transitory computer readable medium and a corresponding computer-program according to the further independent claims.

This Summary lists several embodiments of the presently disclosed subject matter, and in many cases lists variations and permutations of these embodiments. This Summary is merely exemplary of the numerous and varied embodiments. Mention of one or more representative features of a given embodiment is likewise exemplary.

In some embodiments, the ultrasonic energy has a frequency of between about 1 and about 10 megahertz (MHz). In some embodiments, the sequence of ultrasound pulses comprises burst pulses having a frequency of about 1.7 MHz and imaging pulses having a frequency of about 3 MHz. In some embodiments, the ultrasonic energy is applied at a mechanical index ranging from about 0.5 to about 1.9.

In some embodiments, the sequence of ultrasound pulses comprises a series of burst pulse sequences, optionally wherein the duration of each burst pulse sequence is between about 3 and about 5 seconds and the time interval between burst pulse sequences is about 1 second.

In some embodiments, the ultrasonic energy "on" time, relative to the time between successive pulses, lies in the range of about 1% to about 10%. In some embodiments, each burst pulse sequence comprises a series of pulse sub-sequences, optionally wherein each pulse sub-sequence comprises a series of pulse repetitions at a single lateral location.

In some embodiments, the invention protects a subject in need thereof from kidney IRI. In some embodiments, the subject is a human. In some embodiments, the ultrasonic energy is applied about 24 to 48 hours prior to the subject undergoing a surgery that will result in an ischemic event.

According to the invention, the presently disclosed subject matter provides a system for effecting ultrasound-based protection from ischemia/reperfusion injury (IRI) and/or mitigating in the subject an inflammatory condition, disease or disorder, said system comprising: an imaging device for imaging a target tissue region; a transducer for performing a volumetric sweep through the target volume systematically for a selected total time duration and for applying ultrasonic energy to a volume region of interest (ROI), wherein applying the ultrasonic energy comprises emitting a sequence of ultrasonic pulses from the transducer, the sequence of ultrasound pulses having predetermined frequency, mechanical index, pulse length(s), and pulse spacing(s), while performing a volumetric sweep through the ROI systematically; a processor for identifying a volume ROI by highlighting a region on one of more image frames as the transducer is swept through a volume; and a controller for selecting a therapeutic ultrasound dose comprising a predetermined frequency, mechanical index, pulse length, and pulse spacing. The imaging device and the transducer are the same component.

In some embodiments, said transducer is placed in a mechanical translation stage to automatically effect the sweep. In some embodiments, said transducer is configured to track elevation motion to assure that a desired sweep velocity is used. In some embodiments, said transducer is a sector transducer array, optionally wherein it steers to +/- 45 degrees. In some embodiments, said transducer is a curved linear transducer array. In some embodiments, said transducer comprises a 2D array that can sweep through an entire target volume without physical translation.

In some embodiments, said selected total time duration ranges from about 3 minutes to about 15 minutes, optionally wherein said total time duration is about 10 minutes. In some embodiments, the ultrasonic energy has a frequency of between about 1 and about 10 megahertz (MHz). In some embodiments, the sequence of ultrasound pulses comprises burst pulses having a frequency of about 1.7 MHz and imaging pulses having a frequency of about 3 MHz. In some embodiments, the ultrasonic energy is applied at a mechanical index ranging from about 0.5 to about 1.9.

In some embodiments, the sequence of ultrasound pulses comprises a series of burst pulse sequences, optionally wherein the duration of each burst pulse sequence is between about 3 and about 5 seconds and the time interval between burst pulse sequences is about 1 second.

In some embodiments, the ultrasonic energy "on" time, relative to the time between successive pulses, lies in the range of about 1% to about 10%. In some embodiments, each burst pulse sequence comprises a series of pulse sub-sequences, optionally wherein each pulse sub-sequence comprises a series of pulse repetitions at a single lateral location.

In some embodiments, said system uses rates of image decorrelation to estimate rate of elevational motion and to provide feedback as to "too fast" or "too slow". In some embodiments, said system measures instantaneous sweep velocity and provides an audible or visual cue as to "too fast" or "too slow".

According to the invention, said system further comprises an image library database. Said image library data base provides an image comparison/similarity algorithm that is used to automatically identify spleen and thereby identify an optimal ROI that encompasses it.

According to the invention, the presently disclosed subject matter provides a non-transitory computer readable medium having stored thereon executable instructions that when executed by the processor of a computer control the computer to perform steps comprising: imaging a target tissue region within said subject, wherein the target tissue region comprises spleen tissue; identifying a volume region of interest (ROI) in said target tissue; and applying ultrasonic energy to the ROI, wherein applying the ultrasonic energy comprises emitting a sequence of ultrasonic pulses from an ultrasound transducer, the sequence of ultrasound pulses having predetermined frequency, mechanical index, pulse length, and pulse spacing, while performing a volumetric sweep through the ROI systematically for a selected total time duration of between about 3 minutes and about 15 minutes. In some embodiments, the total time duration is about 10 minutes.

In some embodiments, the ultrasonic energy has a frequency of between about 1 and about 10 megahertz (MHz). In some embodiments, the sequence of ultrasound pulses comprises burst pulses having a frequency of about 1.7 MHz and imaging pulses having a frequency of about 3 MHz. In some embodiments, the ultrasonic energy is applied at a mechanical index of ranging from 0.5 to about 1.9.

In some embodiments, the sequence of ultrasound pulses comprises a series of burst pulse sequences, optionally wherein the duration of each burst pulse sequence is between about 3 and about 5 seconds and the time interval between burst pulse sequences is about 1 second.

In some embodiments, the ultrasonic energy "on" time, relative to the time between successive pulses, lies in the range of about 1% to about 10%. In some embodiments, each burst pulse sequence comprises a series of pulse sub-sequences, optionally wherein each pulse sub-sequence comprises a series of pulse repetitions at a single lateral location.

According to the invention, the executable instructions that when executed by the processor of a computer control the computer to interact with an image library database. The executable instructions comprise an image comparison/similarity algorithm that is used to automatically identify spleen and thereby identify an optimal ROI that encompasses it.

An object of the presently disclosed subject matter having been stated hereinabove, and which is achieved in whole or in part by the presently disclosed subject matter, other objects will become evident as the description proceeds hereinbelow.

### BRIEF DESCRIPTION OF THE DRAWINGS

Figure 1 is a schematic representation showing that cholinergic anti-inflammatory pathway is central to various experimental models of inflammatory disease.
Figure 2 is a sample spleen image showing sonographic measurement of the spleen.
Figure 3 is a sample spleen image with a region of interest (ROI) approximately encompassing the target region.
Figure 4 is a schematic representation of an ultrasound system **700** according to an aspect of an embodiment of the presently disclosed subject matter, which is referred to in order to generally describe the operations of an ultrasound system to produce an image of an object **13**.
Figure 5 is a block diagram illustrating an example of a machine **400** upon which one or more aspects of embodiments of the presently disclosed subject matter (e.g., discussed methodologies) can be implemented (e.g., run).
Figure 6 is a schematic diagram of ultrasound (US) sequencing employed in Example 2.
Figure 7 is a schematic diagram of ultrasound (US) sequencing employed in Example 2.
Figures 8A and 8B are plots of a single imaging pulse and of an imaging pulse train, respectively; and Figure 8C is a photograph of a related ultrasound system display. The imaging pulse frequency is 2.8 Megahertz (MHz); the interval between pulses is approximately 200 microseconds (µs); and focal depth is set at 60 millimeters (mm).
Figures 9A and 9B are a plot and a photograph of an ultrasound system display, respectively, showing a single burst pulse. Burst pulse frequency is: 1.7 Megahertz (MHz) and the burst train duration was set to 50 microseconds (µs), continuously repeated at an interval of 1 second (s) using a programmable mode as described in Example 2). The lowest interval possible is 1 s and the lowest duration possible is 50 µs. Interval between pulses is approximately 6 ms.
Figures 10A-10C are a series of photographs of an ultrasound system display showing a burst frame with an interval between A-lines of 6 milliseconds (ms) (Figure 10A); a single burst A-line, with an interval between pulses of 0.3 ms (Figure 10B); and a single burst pulse (Figure 10C).
Figures 11A-11C are a series of plots showing the ultrasound settings employed in Example 2: del T (interval between burst frames) set to 54 milliseconds (ms) (Figure 11A); burst A line pulse interval = 0.3 ms = 3.3 kilohertz (KHz); amplitude variation in burst A line reflects off axis pulses (Figures 11B and 11C).
Figures 12A and 12B are a set of plots showing burst frame trains of varying durations as employed in Example 2 (i.e., 49 milliseconds (ms) in Figure 12A and 147 ms in the three frame burst train of Figure 12B).

### DETAILED DESCRIPTION

The presently disclosed subject matter will now be described more fully. The presently disclosed subject matter can, however, be embodied in different forms, falling within the scope of the independent claims, and should not be construed as limited to the embodiments set forth herein below and in the accompanying Examples. Rather, these embodiments are provided so that this disclosure will be thorough and complete, and will fully convey the scope of the embodiments to those skilled in the art.

Acoustic power is expressed in a variety of ways by those skilled in the art. One method of estimating the acoustic power of an acoustic wave on tissue is the "Mechanical Index". The Mechanical Index (MI) is a standard measure of the acoustic output in a diagnostic ultrasound system, defined as the peak negative pressure, or rarefactional pressure, (in units of MPa - megaPascals) of an ultrasound wave propagating in a uniform medium, divided by the square root of the centre frequency (in units of MHz) of the transmitted ultrasound pulse. The uniform medium is assumed to have an attenuation of 0.3 dB/cm/MHz (attenuation coefficient divided by ultrasound frequency). MI is proportional to pulse voltage and inversely proportional to the square root of the pulse frequency. The mechanical index (MI) is intended to offer an approximate guide to the likelihood of the occurrence of cavitational-related bioeffects. High acoustic pressure ultrasound systems generally have a MI greater than 10. Low acoustic pressure systems generally have a MI lower than 5. For example, diagnostic ultrasound systems are limited by U.S. FDA to a Mechanical Index not to exceed 1.9.

Another measurement used by those skilled in the art is the spatial peak, peak average intensity ("Isppa"). The intensity of an ultrasound beam is greater at the center of its cross section than at the periphery. Similarly, the intensity varies over a given pulse of ultrasound energy. Isppa is measured at the location where intensity is maximum averaged over the pulse duration. Isppa for high acoustic pressure or HIFU applications ranges from approximately 1500 W/cm² to 9000 W/cm². Diagnostic ultrasound equipment, for instance, will generally have, and an Isppa less than 700 W/cm².

Yet another way in which ultrasound waves can be characterized is by the amplitude of their peak negative pressure. "High acoustic pressure" or "HIFU" applications employ waves with peak amplitudes in excess of 10 MPa. "Low acoustic pressure" ultrasound includes ultrasound waves will generally have peak negative pressures in the range of 0.01 to 5.0 MPa. Diagnostic ultrasound equipment, for example, will generally have a peak amplitude less than 3.0 MPa.

Both high and low acoustic pressure ultrasound systems generally operate within the frequency range of 250 KHz-10.0 MHz. Diagnostic imaging typically uses frequencies of about 1.0 MHz to about 5.0 MHz. One known low acoustic pressure ultrasound probe may produce ultrasound having a frequency as high as 7.5 MHz. Some low acoustic pressure ultrasound probes may produce ultrasound frequencies as high as 10.0 MHz. Physical therapy ultrasound systems generally operate at frequencies of either 1.0 MHz or 3.3 MHz.

### II. IMAGE GUIDED ULTRASOUND FOR PROTECTION FROM ISCHEMIA REPERFUSION INJURY

Ultrasound (US) is a tool that can achieve spatial focused delivery of energy. Among its non-thermal properties, US can also modulate frog peripheral nerve activity and stimulate electrical activity in hippocampal neurons by activating voltage-gated ion channels, and transcranial US-induced neuromodulation produced limb movement in rodents. These findings suggest a potentially important, unrecognized, innovative therapeutic approach to human disease based on biomechanical non-thermal effects of US.

In accordance with the presently disclosed subject matter, it has been found that US pulses protect the kidney from ischemia-reperfusion injury (IRI) in an animal model of AKI. Without being bound to any one theory, it is believed that US protection is afforded because US can activate the cholinergic anti-inflammatory pathway (CAP). The CAP serves as an interface between the nervous and immune systems to maintain homeostatic mechanisms to respond to stress or injury and has been implicated as a favorable therapeutic target in numerous diseases/disorders, such as, but not limited to, sepsis, colitis, arthritis, endotoxemia, pancreatitis, hemorrhagic shock, intracerebral hemorrhage, myocarcial infarction (MI), and renal ischemia-reperfusion injury. See Figure 1. The rapid response facilitated by nerve conduction provides immediate signaling to attenuate inflammation. CAP involves the spleen, as it appears to play a role in an inflammatory reflex for the neural control of inflammation and provides a potential therapeutic target for immune-mediated diseases.

In some embodiments, the presently disclosed subject matter provides a method of protecting a subject in need thereof from ischemia/reperfusion injury (IRI). In some embodiments, the method comprises: imaging a target tissue region within said subject; identifying a volume region of interest (ROI) in said target tissue; applying ultrasonic energy to the ROI, wherein applying the ultrasonic energy comprises emitting a sequence of ultrasonic pulses from an ultrasound transducer. In some embodiments, the sequence of ultrasound pulses has a predetermined frequency, mechanical index, pulse lengths, and pulse spacings. In some embodiments, the sequence of ultrasonic pulses is emitted while performing a volumetric sweep through the ROI systematically for a selected total time duration. In some embodiments, the total time duration ranges from about 3 minutes to about 15 minutes. In some embodiments, the total time duration ranges from about 5 minutes to about 15 minutes. In some embodiments, the total time duration is about 10 minutes.

In some embodiments of the presently disclosed subject matter, a largely unmodified diagnostic ultrasound scanner is employed. In some embodiments, an unmodified sector scanning transducer (e.g. Siemens 4V2 or 4V1, Siemens Medical Solutions USA, Inc., Mountain View, California, United States of America, or GE/Philips equivalents, GE Healthcare, Chicago, Illinois, United States of America; Philips, Amsterdam, Netherlands) can be implemented as well. An aspect of an embodiment of the present presently disclosed subject matter provides, but is not limited thereto, a user interface to guide the imaging and therapeutic operations. Initially, the spleen is detected in the ultrasound image and then a volumetric scan sequence is identified.

The target tissue region comprises spleen tissue. Furthermore, in some embodiments of the presently disclosed systems and methods are used, but not limited thereto, with regard to the following clinical applications: therapy for AKI; therapy for protection from IRI post myocardial infarction; therapy for protection from IRI in connection with surgical procedures; therapy for colitis; therapy for endotoxemia/sepsis; therapy for pancreatitis; and therapy for rheumatoid arthritis. In some embodiments, the subject of the presently disclosed methods is typically mammalian. For example, subjects of the presently disclosed subject matter include, but are not limited to, humans and other primates, as well as other mammals including commercially relevant mammals or mammals kept as pets or in zoos, such as cattle, pigs, horses, sheep, cats, dogs, goats, bears, rabbits and rodents. Suitable subjects also include birds, including commercially relevant birds and birds kept as pets, such as chickens, ducks, geese, parrots, and turkeys. In some embodiments, the subject is a human. In some embodiments, the ultrasonic energy is applied about 24 to 48 hours prior to the subject undergoing a surgery that will result in an ischemic event. In some embodiments, the subject is in need of mitigation of an inflammatory condition, disease or disorder, such as but not limited colitis, endotoxemia/sepsis, pancreatitis, or rheumatoid arthritis.

According to some embodiments of the presently disclosed subject matter, a patient is diagnosed or otherwise screened in advance for IRI protection therapy. For example, patients scheduled for certain types of invasive surgeries and patients in the intensive care unit (ICU) with IRI risk (e.g., ICU patients with multi-organ disease and/or sepsis) are identified. B-mode ultrasound is used to find the spleen using well-known and documented anatomical landmarks. As previously described (see, e.g., J Ultrasound Med., 2011, 30:1281-1293), the spleen can be best examined with the patient in a supine or lateral recumbent position, while the patient exhales so that the upper pole of the spleen is not covered by lung tissue, using the 10^{th} and 11^{th} intercoastal spaces as the acoustic window for ultrasound transmission with sonographic examination from the diaphragmatic end to the lower pole, and/or using a curved array transducer with a median frequency of 3 to 5 MHz. A normal adult human spleen is about 10 to 12 centimeters (cm) in length. See J. Ultrasound Med., 2011, 30:1281-1293. The ultrasound operator then determines the optimal format of volumetric scan - i.e. the orientation of the imaging/ therapy plane and how that plane will be translated to encompass a volume ROI.

Using the ultrasound system user interface, the ultrasound operator can enter desired therapy parameters: frequency, pulse intensity, interval, etc. Suitable parameters are described in more detail hereinbelow and also include the following. In some embodiments, the ultrasonic energy has a frequency of between about 1 and about 10 megahertz (MHz). In some embodiments, sequence of ultrasound pulses comprises burst pulses (e.g., having a frequency of about 1.7 MHz) and imaging pulses (e.g., having a frequency of about 3 MHz). In some embodiments, ultrasonic energy is applied at a mechanical index ranging from about 0.5 to about 1.9, such as about 1, 1.2 or 1.7.

In some embodiments, the sequence of ultrasound pulses comprises a series of burst pulse sequences, wherein the duration of each burst pulse sequence is between about 3 and about 5 seconds and the time interval between burst pulse sequences is about 1 second. In some embodiments, each burst pulse sequence comprises a series of pulse sub-sequences. In some embodiments, each pulse sub-sequence can be referred to as a "burst frame". In some embodiments, each pulse sub-sequence has a duration of about 50 microseconds or greater, such as about 54 microseconds. In some embodiments, each pulse sub-sequence has a duration of about 50 milliseconds or greater, such as about 54 milliseconds. In some embodiments, one or more (or each) pulse sub-sequence comprises a series of pulse repetitions at a single lateral location (e.g., within the ROI).

Other forms of pulse design are possible. For example, in some embodiments, the ultrasonic energy "on" time, relative to the time between successive pulses, lies in the range of about 1% to about 10%. That is, pulses can have an "on" time, relative to pulse interval (i.e. "duty cycle"), lying in the range of 1% to 10%, e.g. 1%, 2%, 3%, 4%, 5%, 6%, 7%, 8%, 9%, or 10%. For example, a 5 cycle 2 MHz pulse is 2.5 microseconds long. At 1% duty cycle, the inter pulse interval is 250 microseconds. At 10% duty cycle, the inter pulse interval is 25 microseconds.

The ultrasound operator can also define a range of motion to fully encompass the spleen. For example, if the spleen fits entirely within the image plane of the transducer, then the transducer can be translated, or tilted, to encompass the 3D spleen volume. If the spleen does not fit within the image plane of the transducer there are two solutions. First, the transducer can be rotated so as to acquire a "short axis" as opposed to a "long axis". Once the short axis is acquired, the transducer can be swept elevationally to encompass the 3D spleen volume. Alternatively, the ultrasound operator can select a transducer with a larger field of view. Typically, a large curved linear transducer will have the greatest near field and far field image dimension and can practically assure that the spleen can be fully encompassed within a volumetric sweep.

Next, the ultrasound operator can perform a "dry run". In the "dry run," the ultrasound machine, in diagnostic imaging mode only, scans across the tissue region and color codes (translucent overlay) the region that would be insonified with a "live" therapy insonification. The scanner produces a three dimensional (3D) representation of the anatomy and the color overlay. If the color overlay correctly insonifies all the tissue required, the ultrasound operator hits a "go" button and therapy is administered. If the overlap is incorrect, the operator modifies the scanning orientation and position and repeats the process until correct overlay is obtained and a "live" procedure can be conducted.

The volumetric scan involves sweeping the transducer through a volume - i.e. stepping the transducer gradually in the direction orthogonal to the imaging plane. The volumetric scan can use a computer controlled motion stage or some form of 3D positioning system that allows for freehand sweep. Freehand sweeping can be performed using optical tracking, magnetic tracking, or image registration-based tracking. Image registration-based tracking can be convenient because it is unobtrusive, but does involve a minor transducer modification, such as, but not limited to, the "I-Beam" approach described in Hossack et al., IEEE Transactions on Ultrasonics Ferroelectrics & Frequency Control, 2002, 49(8):1029-1038. The "I-Beam" has a central (conventional) imaging array. Two perpendicularly oriented "tracking" arrays are attached to either end of the central imaging array (so as to form a shape "I".) The "tracking" arrays are also imaging arrays - forming images perpendicular to that obtained by the central array. As the central array image plane is swept across a volume it collects a sequence of 2D image slices arranged in a "breadslice" manner. By tracking the motion increments detected on the "tracking" array acquired images (e.g. by using a conventional pixel patch matching algorithm - correlation based for example), the offsets between successive acquired images from the central image array - forming the "breadslice" - can be precisely located in 3D space. Once the data is acquired for a sequence of detected location planes, the volumetric data may be interpolated onto a regular 3D grade data set for easier subsequent manipulation. When using a freehand approach, the tracking image motion detection provides the system with scan speed (i.e. mm/s). Therefore, the system user interface will provide real-time feedback to the user - i.e. to "speed up" or "slow down" sweeping, optionally using a graphical "bull's eye" that the user keeps aligned to maintain the optimal scan speed.

However, it also appears that the total energy deposition to the target tissue influences the success of the treatment. Accordingly, in some embodiments, the ultrasound operator can sweep the transducer back and forth across the tissue target at a steady speed multiple times until the total time duration target has been met. For example, in early studies, it appears that a total time duration of about 15 minutes can be employed. However, the total time duration can be a function of: instantaneous ultrasound intensity and pulse "duty factor" - i.e. the proportion of elapsed time during which ultrasound is actually "on". In some embodiments, as described hereinabove, the total time is between about 5 minutes and about 15 minutes (i.e., about 5, 6, 7, 8, 9, 10, 11, 12, 13, 14, or 15 minutes).

In some embodiments, a Verasonics programmable scanner (V-1 or Vantage (Verasonics Inc., Kirkland, Washington, United States of America) or another equivalent scanner can be programed to perform standard B-Mode imaging using any standard beamforming approach. Programming B-Mode, or other similar modes, for imaging falls within the knowledge of those "skilled in the art", upon a review of the instant disclosure.

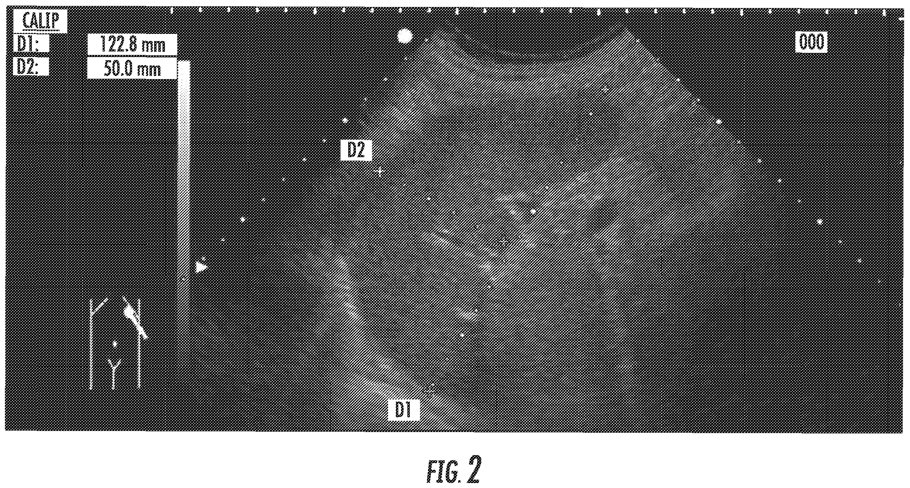

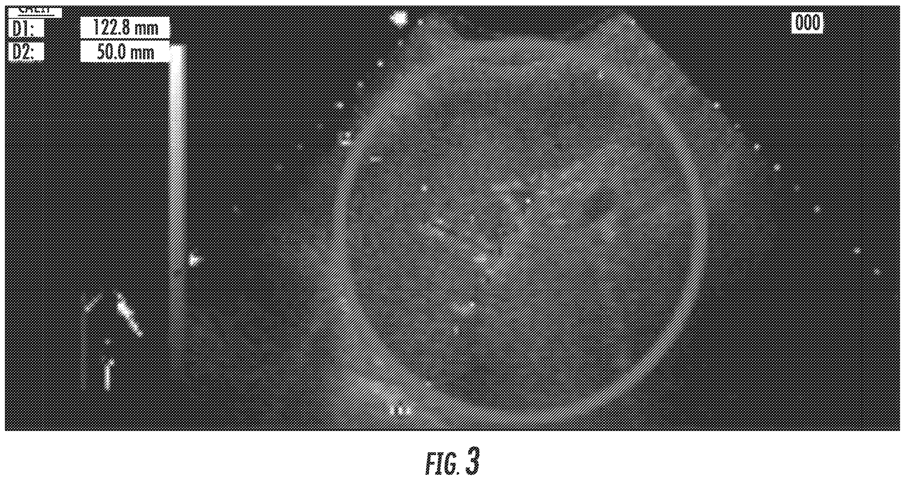

Therapeutic ultrasound can be performed, for example, using the same scanner (e.g., a Verasonics programmable scanner (Verasonics Inc., Kirkland, Washington, United States of America). The scanner can be used to create a user selectable menu of therapeutic protocols. As one example, the therapeutic ultrasound can be applied at about 3 MHz (range 1-10 MHz), Mechanical Index (MI) = 1.2 (range 0.2 to 1.8), 6 (range 2-10) cycles, 0.2 ms (range 0.1 to 0.5 ms) intervals. A user programmed ROI can be selected that sets both the focal depth (middle of the ROI) and the width of the scan in terms of the number of lines required to encompass the ROI from side to side (laterally). See Figures 2 and 3, for example.

Accordingly, in some embodiments, user selectable options for the therapeutic ultrasound include: frequency, the number of pulse cycles per burst, the MI, pulse interval, the focal depth, and the width of the focal zone. More particularly, in some embodiments, the frequency can be between about 1 MHz and about 10 MHz. In some embodiments, the frequency is about 1.7 MHz or about 3 MHz. In some embodiments, the number of cycles per burst can range from 2 to about 10 (e.g., 2, 3, 4, 5, 6, 7, 8, 9, or 10. In some embodiments, the number of cycles per burst can be 6. The MI can be between 0.2 to about 1.8 or 1.9 (where MI = 1.9 is the FDA diagnostic ultrasound limit. In some embodiments, the MI can range from about 0.5 to about 1.9. In some embodiments, the MI is about 1 or higher, or such as about 1.2 or higher. In some embodiments, the pulse interval between pulses within burst patterns is between about 0.1 and about 0.5 ms. The focal depth can typically be the center of an ROI that encompasses the spleen. The focal zone width can be between about 10 and about 50 mm, e.g., encompassing the spleen in the ROI.

Additional user selectable options can include, but are not limited to, performing multiple repetitions per beam line. In B-Mode, successive line firings are typically on successive lateral locations, i.e., to sweep laterally across the field of view. However, in some embodiments, multiple firings along the same line can induce the desired therapeutic effect more efficiently. For example, in some embodiments, about 4 to about 10 beam repetitions can be performed at a single lateral location before stepping to the next location.

An aspect of an embodiment of the presently disclosed system (and method) provides for effecting therapeutic ultrasound, such as ultrasound-based protection from IRI, that can comprise, among other things, the sequence of:
a) imaging a target tissue region using ultrasound imaging;
b) identifying a volume region of interest (ROI) by highlighting a region on one of more ultrasound image frames as an ultrasound transducer is swept through a volume,
c) selecting a therapeutic ultrasound dose comprising a selected intensity, pulse length and/or pulse spacing; and
d) performing a volumetric sweep through the ROI systematically for a selected total time duration (for example, in the range 5 to 15 minutes).

Furthermore, an aspect of an embodiment of the presently disclosed system (and related method) can include, but is not limited to: a mechanical translation stage into which the ultrasound transducer can be placed to automatically effect the sweep; a transducer that is configured to track elevation motion to assure that a desired sweep velocity is used, such as by using "I-Beam"; a system that uses rates of image decorrelation to estimate rate of elevational motion and to provide feedback as to "too fast or too slow", a transducer comprising a sector transducer array (i.e., that steers to +/- 45 degrees), a transducer that comprises a curved linear transducer array, a transducer that comprises a two dimensional (2D) array that is configured to sweep through an entire target volume without physical translation; an ultrasound system configured to measure instantaneous sweep velocity and provide an audible or visual cue as to "too fast" or "too slow", and/or a system that comprises an image library and/or the performance of image comparison/a similarity method to automatically identify the subject's spleen and thereby identify an optimal ROI that encompasses it.

Moreover, an aspect of an embodiment of the presently disclosed subject matter can be applicable to a variety of different modes of use. In some embodiments, a physician or other heath care professional can identify a subject for the presently disclosed methods via a non-imaging test (e.g., a blood test, such as a blood test performed post MI, etc.). In some embodiments, a physician or other health care professional can identify a subject based on the subject being scheduled to have surgery that can cause IRI. In some embodiments, the physician, other heath care provider and/or an ultrasound operator can identify the subject's spleen during imaging and immediately initiate a therapeutic ultrasound sweep. In some embodiments, a physician or other health care professional can initiate an imaging study and therapeutic sweep and direct successive sweeps on a daily, weekly or monthly basis.

In some embodiments, contrast microbubbles can be infused prior to ultrasound to highlight and differentiate the spleen. In some embodiments, the ultrasound focal conditions can be modified, e.g., to time serial beams with progressively deeper foci (e.g., similar to multiple transmit focus in B-Mode ultrasound) or to spread focal depth beams (e.g., to use "axicon" beams).

It should be appreciated that a variety of ultrasound related systems and methods can be utilized as part of implementing or practicing aspects of the various embodiments of the presently disclosed subject matter.

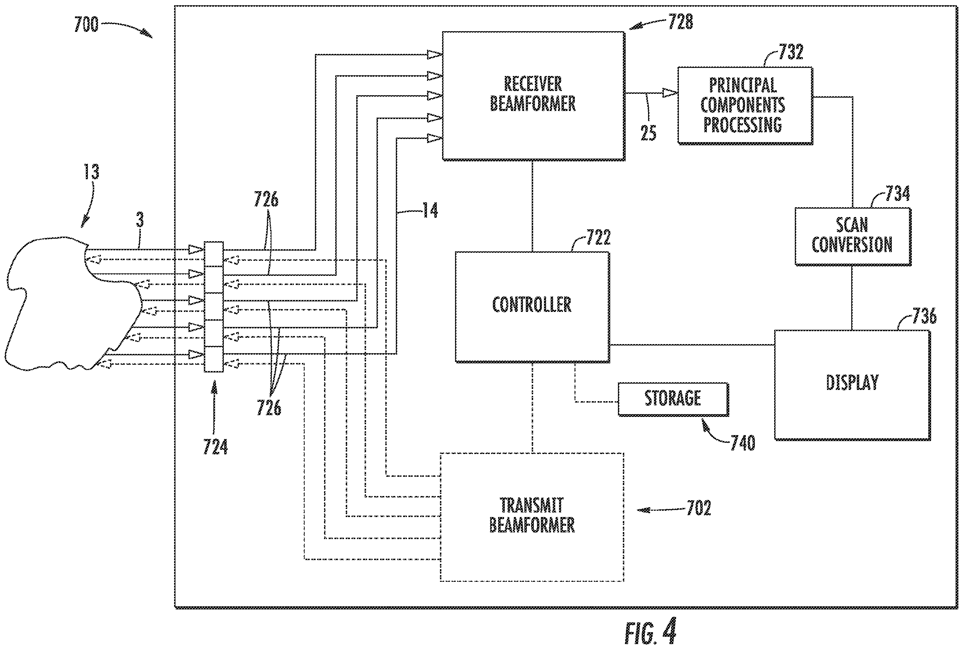

Figure 4 is a basic, schematic representation of an ultrasound system **700** according to an aspect of an embodiment of the presently disclosed subject matter that is referred to in order to generally describe the operations of an ultrasound system to produce an image of an object **13** (i.e the spleen inside a subject). System **700** can optionally include a transmit beamformer **702** which can include input thereto by controller **722** to send electrical instructions to array **724** (which can also be referred to as a transducer or a probe) as to the specifics of the ultrasonic waves to be emitted by array **724**. Alternatively, system **700** can be a receive-only system and the emitted waves may be directed to the object **13** from an external source.

In either case, echoes **3** reflected by the object **13** (and surrounding environment) are received by array **724** and converted to electrical (e.g., radio frequency (RF)) signals **726** that are input to receive beamformer **728**. Controller **722** can be external of the beamformer **728**, as shown, or integrated therewith. Controller **722** can automatically and dynamically change the distances at which scan lines are performed (when a transmit beamformer **702** is included) and automatically and dynamically controls the receive beamformer **728** to receive signal data for scan lines at predetermined distances. Distance/depth is typically calculated assuming a constant speed of sound in tissue (e.g., 1540 m/s or as desired or required) and then time of flight is recorded such that the returning echoes have a known origination. The summed RF lines output by the receive beamformer **728** are input to a principal components processing module **732**, which may be separate from and controlled by, or incorporated in controller **722**. Principal components module **732** processes received echo data. The assembled output can be input into a scan converter module **734**. The image formed within the scan converter **734** is displayed on display **736**.

Controller **722** can have incorporated therein an input unit (e.g., comprising, for example, a keyboard, a mouse, a trackball, a tablet or a touch screen module) for the ultrasound operator to input control commands related to the operation of system **700.** Control commands that can be input into controller **722** can include specifics of the ultrasonic waves emitted by transducer **724**, such as, but not limited to, pulse frequency, pulse interval, etc. Alternatively, controller **722** and/or system **700** can be in communication with a machine **400** as describe further herein below.

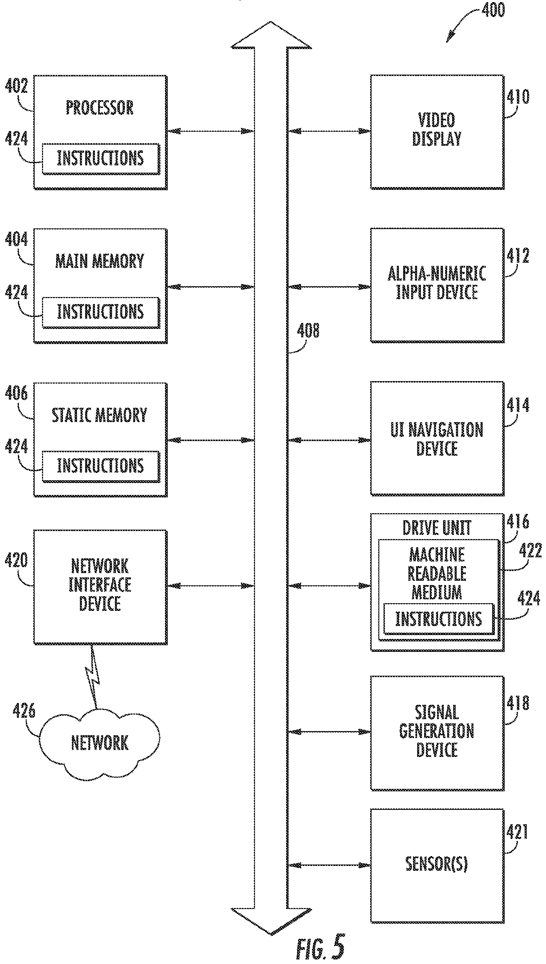

Transducer **724** is shown as an array in Figure 5. Transducer **724** can be attached to the remainder of system **700** via a cable and be independently translatable, e.g., so that an ultrasound operator can manually translate transducer **724** over the skin surface of a subject to locate internal object **13** (e.g., spleen or another organ or tissue of interest). Alternatively, transducer **724** can be placed in an optional mechanical translation stage (e.g., controlled by controller **722**) to automatically and systematically effect a sweep of particular volume of object **13**. Transducer **724** can be a sector transducer array that steers to +/- 45 degrees. Transducer **724** can be a curved linear array or a two dimensional (2D) array that can sweep an entire target volume of object **13** without physical translation. Further, although Figure 5 has been described as an ultrasound system, it is noted that transducer **724** can alternatively be transducers for converting electrical energy to forms of energy other than ultrasound and vice versa, including, but not limited to radio waves (e.g., where system **700** is configured for RADAR), visible light, infrared, ultraviolet, and/or other forms of sonic energy waves, including, but not limited to SONAR, or some other arbitrary signal of arbitrary dimensions greater than one (such as, for example, a signal that is emitted by a target).

System **700** further includes storage **740** which can store an image of object **13** (e.g., a previously acquired ultrasound image, or a computed tomography (CT) image acquired by an external CT scanner or a magnetic resonance (MR) image of object **13** obtained by an external magnetic resonance imaging (MRI) device. The previously acquired image can be a volume image of object **13** which includes volume information of object **13**. According to the invention, storage **740** can include an image library database. The image library database provides an image comparison/similarity algorithm to automatically identify spleen and, thereby, identify an optimal target region of interest (ROI) that encompasses the spleen.

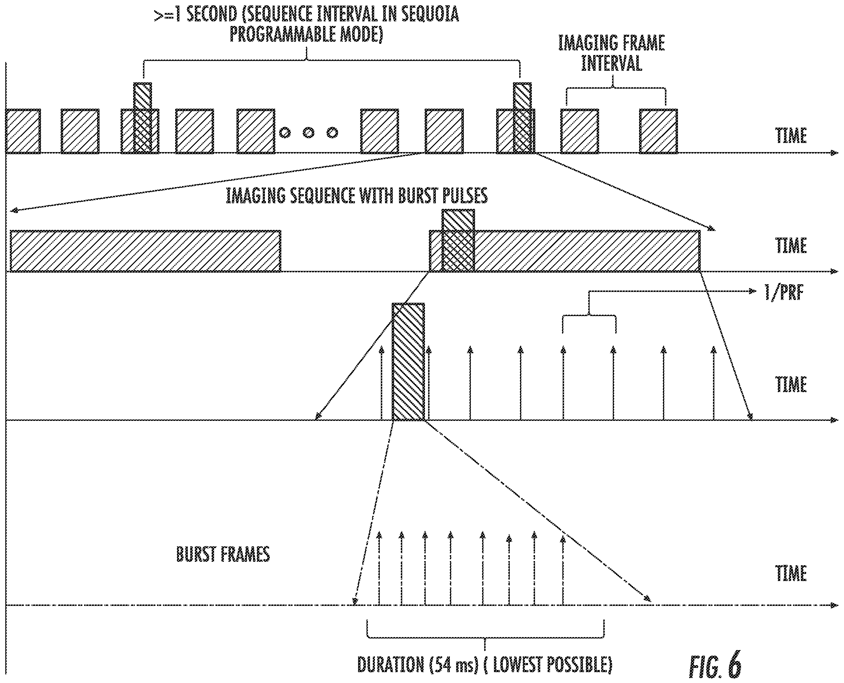

Figure 6 is a block diagram illustrating an example of a machine upon which one or more aspects of embodiments of the presently disclosed subject matter can be implemented. Figure 6 illustrates a block diagram of an example machine **400** upon which one or more embodiments (e.g., discussed methodologies) can be implemented (e.g., run).

Examples of machine **400** can include logic, one or more components, circuits (e.g., modules), or mechanisms. Circuits are tangible entities configured to perform certain operations. In an example, circuits can be arranged (e.g., internally or with respect to external entities such as other circuits) in a specified manner. In an example, one or more computer systems (e.g., a standalone, client or server computer system) or one or more hardware processors (processors) can be configured by software (e.g., instructions, an application portion, or an application) as a circuit that operates to perform certain operations as described herein. In an example, the software can reside (1) on a non-transitory machine readable medium or (2) in a transmission signal. In an example, the software, when executed by the underlying hardware of the circuit, causes the circuit to perform the certain operations.

In an example, a circuit can be implemented mechanically or electronically. For example, a circuit can comprise dedicated circuitry or logic that is specifically configured to perform one or more techniques such as discussed above, such as including a special-purpose processor, a field programmable gate array (FPGA) or an application-specific integrated circuit (ASIC). In an example, a circuit can comprise programmable logic (e.g., circuitry, as encompassed within a general-purpose processor or other programmable processor) that can be temporarily configured (e.g., by software) to perform the certain operations. It will be appreciated that the decision to implement a circuit mechanically (e.g., in dedicated and permanently configured circuitry), or in temporarily configured circuitry (e.g., configured by software) can be driven by cost and time considerations.

Accordingly, the term "circuit" is understood to encompass a tangible entity, be that an entity that is physically constructed, permanently configured (e.g., hardwired), or temporarily (e.g., transitorily) configured (e.g., programmed) to operate in a specified manner or to perform specified operations. In an example, given a plurality of temporarily configured circuits, each of the circuits need not be configured or instantiated at any one instance in time. For example, where the circuits comprise a general-purpose processor configured via software, the general-purpose processor can be configured as respective different circuits at different times. Software can accordingly configure a processor, for example, to constitute a particular circuit at one instance of time and to constitute a different circuit at a different instance of time.

In an example, circuits can provide information to, and receive information from, other circuits. In this example, the circuits can be regarded as being communicatively coupled to one or more other circuits. Where multiple of such circuits exist contemporaneously, communications can be achieved through signal transmission (e.g., over appropriate circuits and buses) that connect the circuits. In embodiments in which multiple circuits are configured or instantiated at different times, communications between such circuits can be achieved, for example, through the storage and retrieval of information in memory structures to which the multiple circuits have access. For example, one circuit can perform an operation and store the output of that operation in a memory device to which it is communicatively coupled. A further circuit can then, at a later time, access the memory device to retrieve and process the stored output. In an example, circuits can be configured to initiate or receive communications with input or output devices and can operate on a resource (e.g., a collection of information).

The various operations of method examples described herein is performed by one or more processors that are temporarily configured (e.g., by software) or permanently configured to perform the relevant operations. Whether temporarily or permanently configured, such processors can constitute processor-implemented circuits that operate to perform one or more operations or functions. In an example, the circuits referred to herein can comprise processor-implemented circuits.

In some embodiments, the processor or processors can be located in a single location (e.g., within a home environment, an office environment or as a server farm), while in other examples the processors can be distributed across a number of locations.

The one or more processors can also operate to support performance of the relevant operations in a "cloud computing" environment or as a "software as a service" (SaaS). For example, at least some of the operations can be performed by a group of computers (as examples of machines including processors), with these operations being accessible via a network (e.g., the Internet) and via one or more appropriate interfaces (e.g., Application Program Interfaces (APIs).)

Exemplary embodiments (e.g., apparatus, systems, or methods) can be implemented in digital electronic circuitry, in computer hardware, in firmware, in software, or in any combination thereof. Exemplary embodiments can be implemented using a computer program product (e.g., a computer program, tangibly embodied in an information carrier or in a machine readable medium, for execution by, or to control the operation of, data processing apparatus such as a programmable processor, a computer, or multiple computers).

A computer program can be written in any form of programming language, including compiled or interpreted languages, and it can be deployed in any form, including as a stand-alone program or as a software module, subroutine, or other unit suitable for use in a computing environment. A computer program can be deployed to be executed on one computer or on multiple computers at one site or distributed across multiple sites and interconnected by a communication network.

In an example, operations can be performed by one or more programmable processors executing a computer program to perform functions by operating on input data and generating output. Examples of method operations can also be performed by, and example apparatus can be implemented as, special purpose logic circuitry (e.g., a field programmable gate array (FPGA) or an application-specific integrated circuit (ASIC)).

The computing system can include clients and servers. A client and server are generally remote from each other and generally interact through a communication network. The relationship of client and server arises by virtue of computer programs running on the respective computers and having a client-server relationship to each other. In embodiments deploying a programmable computing system, it will be appreciated that both hardware and software architectures require consideration. Specifically, it will be appreciated that the choice of whether to implement certain functionality in permanently configured hardware (e.g., an ASIC), in temporarily configured hardware (e.g., a combination of software and a programmable processor), or a combination of permanently and temporarily configured hardware can be a design choice. Below are set out hardware (e.g., machine **400**) and software architectures that can be deployed in example embodiments.

In an example, the machine **400** can operate as a standalone device or the machine **400** can be connected (e.g., networked) to other machines.

In a networked deployment, machine **400** can operate in the capacity of either a server or a client machine in server-client network environments. In an example, machine **400** can act as a peer machine in peer-to-peer (or other distributed) network environments. The machine **400** can be a personal computer (PC), a tablet PC, a set-top box (STB), a Personal Digital Assistant (PDA), a mobile telephone, a web appliance, a network router, switch or bridge, or any machine capable of executing instructions (sequential or otherwise) specifying actions to be taken (e.g., performed) by the machine **400**. Further, while only a single machine **400** is illustrated, the term "machine" shall also be taken to include any collection of machines that individually or jointly execute a set (or multiple sets) of instructions to perform any one or more of the methodologies discussed herein.

Example machine (e.g., computer system) **400** can include a processor **402** (e.g., a central processing unit (CPU), a graphics processing unit (GPU) or both), a main memory **404** and a static memory **406**, some or all of which can communicate with each other via a bus **408**. The machine **400** can further include a display unit **410**, an alphanumeric input device **412** (e.g., a keyboard), and a user interface (Ul) navigation device **411** (e.g., a mouse). In an example, the display unit**410**, input device **412** and UI navigation device **414** can be a touch screen display. The machine **400** can additionally include a storage device (e.g., drive unit) **416**, a signal generation device **418** (e.g., a speaker), a network interface device **420**, and one or more sensors **421**, such as a global positioning system (GPS) sensor, compass, accelerometer, or other sensor.

The storage device **416** can include a machine readable medium **422** on which is stored one or more sets of data structures or instructions **424** (e.g., software) embodying or utilized by any one or more of the methodologies or functions described herein. The instructions **424** can also reside, completely or at least partially, within the main memory **404**, within static memory **406**, or within the processor **402** during execution thereof by the machine **400**. In an example, one or any combination of the processor **402**, the main memory **404**, the static memory **406**, or the storage device **416** can constitute machine readable media.

While the machine readable medium **422** is illustrated as a single medium, the term "machine readable medium" can include a single medium or multiple media (e.g., a centralized or distributed database, and/or associated caches and servers) that configured to store the one or more instructions **424**. The term "machine readable medium" can also be taken to include any tangible medium that is capable of storing, encoding, or carrying instructions for execution by the machine and that cause the machine to perform any one or more of the methodologies of the present disclosure or that is capable of storing, encoding or carrying data structures utilized by or associated with such instructions. The term "machine readable medium" can accordingly be taken to include, but not be limited to, solid-state memories, and optical and magnetic media. Specific examples of machine readable media can include non-volatile memory, including, by way of example, semiconductor memory devices (e.g., Electrically Programmable Read-Only Memory (EPROM), Electrically Erasable Programmable Read-Only Memory (EEPROM)) and flash memory devices; magnetic disks such as internal hard disks and removable disks; magneto-optical disks; and CD-ROM and DVD-ROM disks.

The instructions **424** can further be transmitted or received over a communications network **426** using a transmission medium via the network interface device **420** utilizing any one of a number of transfer protocols (e.g., frame relay, IP, TCP, UDP, HTTP, etc.). Example communication networks can include a local area network (LAN), a wide area network (WAN), a packet data network (e.g., the Internet), mobile telephone networks (e.g., cellular networks), Plain Old Telephone (POTS) networks, and wireless data networks (e.g., IEEE 802.11 standards family known as Wi-Fi^{®}, IEEE 802.16 standards family known as WiMax^{®}), peer-to-peer (P2P) networks, among others. The term "transmission medium" shall be taken to include any intangible medium that is capable of storing, encoding or carrying instructions for execution by the machine, and includes digital or analog communications signals or other intangible medium to facilitate communication of such software.

### III. EXAMPLES

### EXAMPLE 1

### Initial US Studies in Pigs

An initial study to determine the effects of US pre-treatment on IRI caused by kidney ischemia was performed in a group of 15 pigs (5 controls). Kidney function after reperfusion was assessed by measuring plasma creatinine as a marker of impaired kidney function. The plasma creatinine concentration was reduced by an average of 47% in the US group vs. the control (no US) group using non-optimized instrumentation and methods.

### EXAMPLE 2

### US Therapeutic Procedure Using Diagnostic US System

An ACUSON SEQUOIA^{™} 512 ultrasound machine (Siemens Medical Solutions USA, Inc., Mountain View, California, United States of America) was used to image and treat pigs (40-70 kg outbreed females) with US to determine the efficacy of US treatment on kidney IRI after 90 minutes of ischemia. To image the spleen, a 4V1 transducer was used and set at a frequency of 3 MHz (Plain, not Harmonic). Alpha cv CPS vascular can be chosen form the presets. Frame rate was reduced to as low as possible (e.g., 54 ms/19 Hz using the SEQUOIA^{™} machine). Focal depth can be adjusted using the keyboard to focus on the center of the spleen (approximately 40 mm). Burst duration was set for between 3 and 5 seconds, with 1 second between bursts. A stop watch was used to monitor time. Start burst was hit according to the protocol (every ten seconds for pig). Burst and imaging MI can be adjusted as needed. Both sagittal and transverse sections can be imaged.

Pigs can be continuously scanned along the long or short axes of the spleen, i.e., a sweep side to side (elevation) to encompass the target volume. The orientation of the transducer is not critical. The spleen was verified at autopsy to confirm that the target on the ultrasound system screen was the spleen.

The US procedure was also preformed using a Verasonics P4-2v transducer (Verasonics Inc., Kirkland, Washington, United States of America) transmitting at a frequency of 1.7 MHz during burst and at 3 MHz during imaging. A pulse sequence was programmed for a 3 second burst (with an interval between burst pulses of 6 ms and a burst A-line duration of 50 ms), with 1 second interval (steered through 128 angles (-45 to 45 degrees) due to small aperture of the transducer). During treatment the transducer was held at a specific location for 10 minutes. MI measured in water was 1.7 (in water) at 6 cm (focal distance was adjusted as spleen is more superficial). Treatment was manually stopped after 10 minutes by stopping the sequence.

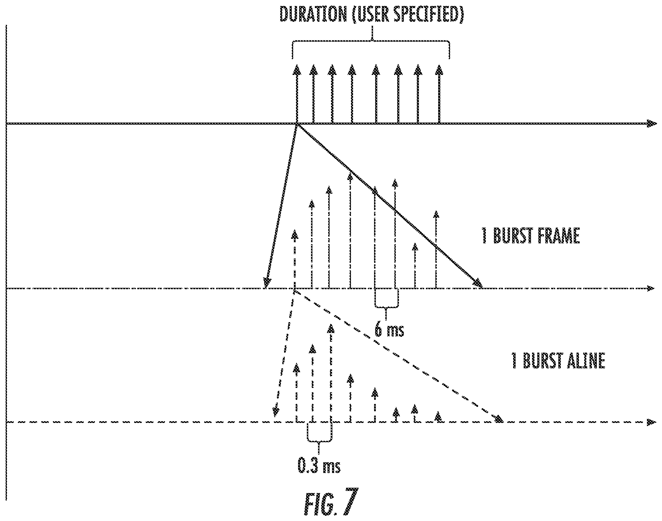

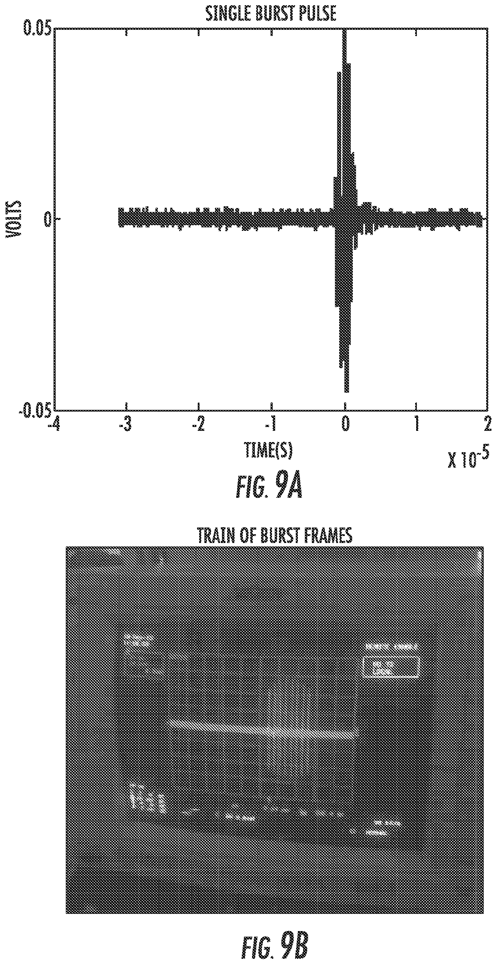

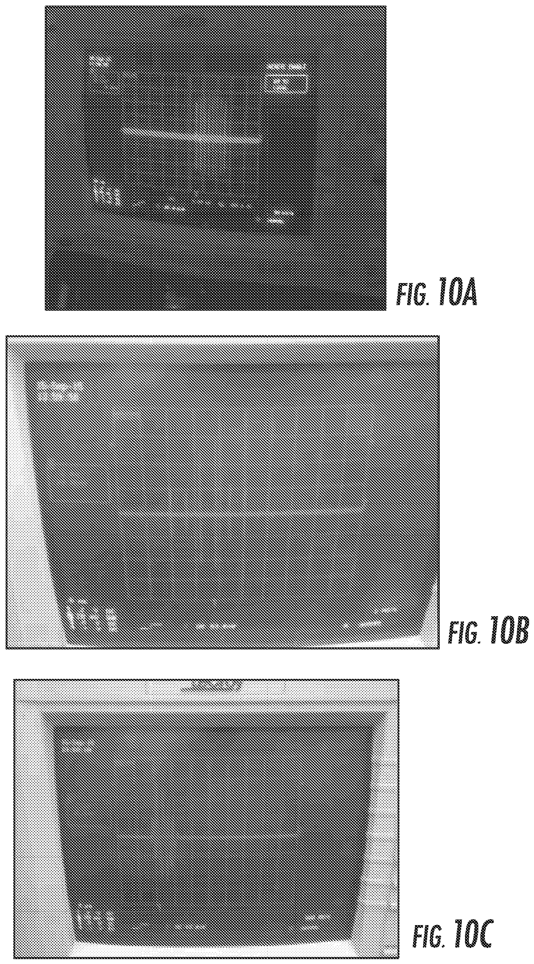

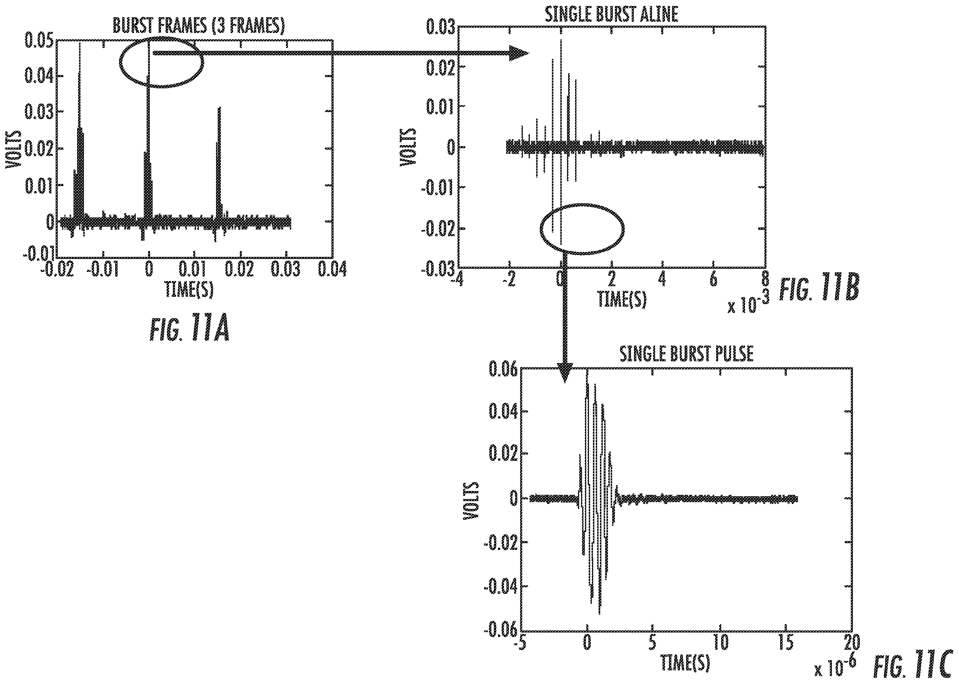

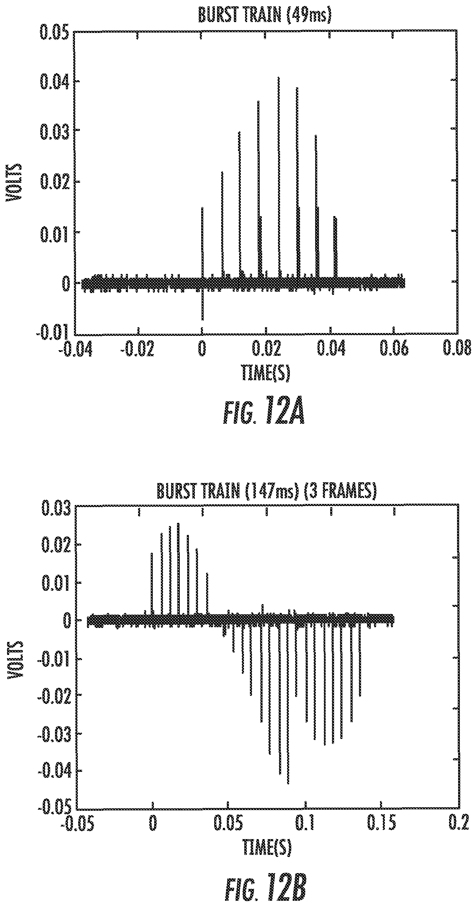

Additional details with regard to pulse sequences can be seen in Figures 6 and 7. A representative pulse sequence included burst pulses and imaging pulses. A set of single burst pulses can be referred to as a burst A-line, while a set of burst A-lines can be referred to as a Burst frame (e.g., 1 execution of a Burst sequence). See Figure 7. The duration of the burst frame was programmed to be 54 ms. See Figure 6. A single burst pulse comprises a 5 cycle pulse at 1.7 MHz. See Figures 9A, 10C, and 11C. Figures 10B and 11B show a single burst A-line with an interval between burst pulses of 0.3 ms. In Figure 11B, the amplitude variation reflects off axis pulses. The interval between burst A-lines was 6 ms. See Figure 10A. Multiple burst frames were performed sequentially to provide a burst train of a user specified duration, with a programmable interval (del T) between frames. See Figures 9B, 11A and 12B. Alternatively, a burst train can comprise a single burst frame. See Figures 10A and 12A. With the 54 ms burst frame duration, there is an upper bound of 9 A-lines in a burst frame and 20 pulses in 1 A-line. All A-lines and pulses are not picked up by the hydrophone because of off axis transmit.

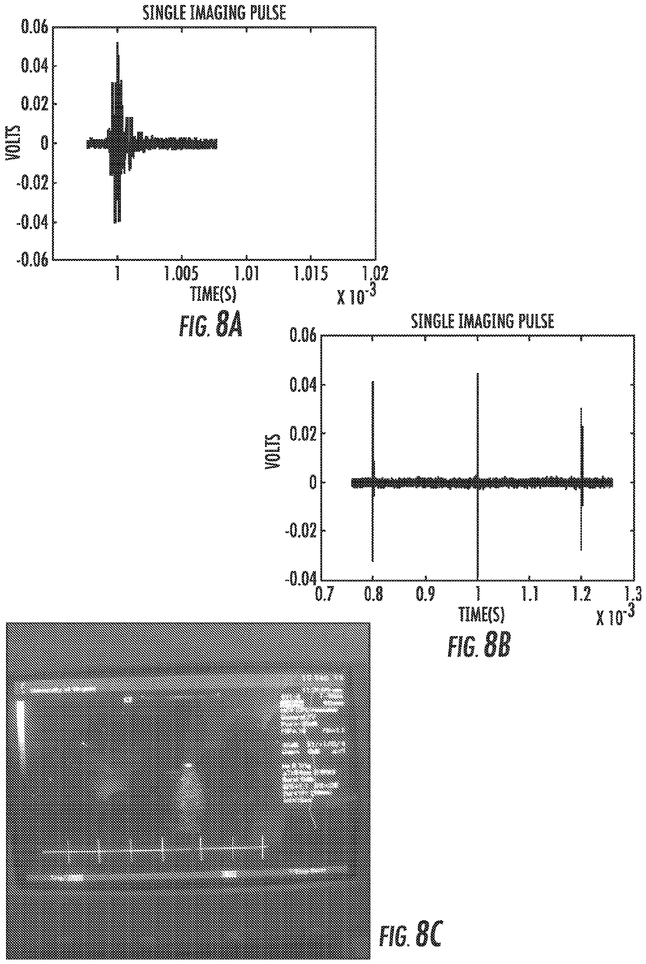

Figures 8A and 8B show typical imaging pulses and imaging pulse trains, while Figure 8C shows a typical US image of a spleen from a single imaging pulse train with an imaging pulse at 2.8 MHz, an interval between pulses of 200 microseconds, a frame rate of 50 Hz and a focal depth of 60 mm.

### IV. REFERENCES

The following patents, applications and publications as listed below and throughout this document.
1. FUSF,http://www.fusfoundation.org/the-technology/manufacturers/manufacturers/list. 2015.
2. Gigliotti, J.C., et al., Ultrasound prevents renal ischemia-reperfusion injury by stimulating the splenic cholinergic anti-inflammatory pathway. J Am Soc Nephrol, 2013. 24(9): p. 1451-60.
3. Gigliotti, J.C., et al., Ultrasound Modulates the Splenic Neuroimmune Axis in Attenuating AKI. J Am Soc Nephrol, 2015.
4. Hossack, J.A., et al., Quantitative 3D Diagnostic Ultrasound Imaging Using a Modified Transducer Array and an Automated Image Tracking Technique. IEEE Transactions on Ultrasonics Ferroelectrics & Frequency Control, 2002. 49(8): p. 1029-1038.
5. U.S. Patent No. 7,617,005 B2, Demarais, et al., "Methods and Apparatus for Thermally-Induced Renal Neuromodulation", November 10, 2009.
6. U.S. Patent No. 7,717,948 B2, Demarais, et al., "Methods and Apparatus for Thermally-Induced Renal Neuromodulation", May 18, 2010.
7. U.S. Patent No. 9,186,198 B2, Demarais, et al., "Ultrasound Apparatuses for Thermally-Induced Renal Neuromodulation and Associated Systems and Methods", November 17, 2015.

Additional references from the prior art:
A. International Patent Application Publication No. WO 2003/075769, Walker, et al., An Intuitive Ultrasonic Imaging System and Related Method Thereof, September 18, 2003.
B. U.S. Patent No. 7,699,776, Walker, et al., Intuitive Ultrasonic Imaging System and Related Method Thereof, issued April 20, 2010.
C. U.S. Patent Application Publication No. US 2010/0268086, Walker, et al., Intuitive Ultrasonic Imaging System and Related Method Thereof, October 21, 2010.
D. U.S. Patent Application Publication No. US 2015/0011884, Walker, et al., Intuitive Ultrasonic Imaging System and Related Method Thereof, January 8, 2015.
E. International Patent Application Serial No. PCT/US2004/000888, Blalock, et al., Ultrasonic Transducer Drive, filed January 14, 2004.
F. U.S. Patent No. 9,244,160, Blalock, et al., Ultrasonic Transducer Drive, issued January 26, 2016.
G. U.S. Patent Application Serial No. 15,005,565, Blalock, et al., Ultrasonic Transducer Drive, filed January 25, 2016.
H. International Patent Application Serial No. PCT/US2004/000887, Walker, et al., Ultrasound Imaging Beam-Former Apparatus and Method, filed January 14, 2004.
I. U.S. Patent Application Publication No. US2007/0016022, Blalock, et al., Ultrasound Imaging Beam-Former Apparatus and Method, January 18, 2007.
J. U.S. Patent No. 9,275,630, Blalock, et al., Ultrasound Imaging Beam-Former Apparatus and Method, issued March 1, 2016.
K. International Patent Application Publication WO 04065978, Hossack, et al., Efficient Ultrasound System for Two-Dimensional C-Scan Imaging and Related Method Thereof, August 5, 2004.
L. U.S. Patent No. 7,402,136, Hossack, et al., Efficient Ultrasound System for Two-Dimensional C-Scan Imaging and Related Method Thereof, issued July 22, 2008.
M. International Patent Application Publication No. WO 060412067, Hossack, et al., Efficient Architecture for 3D and Planar Ultrasonic Imaging - Synthetic Axial Acquisition and Method Thereof, April 20, 2006.
N. U.S. Patent No. 8,057,392, Hossack, et al., Efficient Architecture for 3D and Planar Ultrasonic Imaging - Synthetic Axial Acquisition and Method Thereof, issued November 15, 2011.
O. U.S. Patent Application Publication No. US 2012/0029356, Hossack, et al., Efficient Architecture for 3D and Planar Ultrasonic Imaging - Synthetic Axial Acquisition and Method Thereof, February 2, 2012.
P. U.S. Patent Application Publication No. US 2015/0025387, Hossack, et al., Efficient Architecture for 3D and Planar Ultrasonic Imaging - Synthetic Axial Acquisition and Method Thereof, January 22, 2015.
Q. International Patent Application Publication No. WO 2008/154632, Garson, et al., System and Method for Combined ECG-Echo for Cardiac Diagnosis, December 18, 2008.
R. U.S. Patent Application Publication No. US 2010/0168578, Garson, et al., System and Method for Combined ECG-Echo for Cardiac Diagnosis, July 1, 2010.
S. U.S. Patent No. 7,750,537, Hossack, et al., Hybrid Dual Layer Diagnostic Ultrasound Transducer Array, issued July 6, 2010.
T. U.S. Patent No. 8,093,782, Hossack, Specialized, High Performance, Ultrasound Transducer Substrates and Related Method Thereof, issued January 10, 2012.
U. U.S. Patent Application No. 13/329,965, Hossack, Specialized, High Performance, Ultrasound Transducer Substrates and Related Method Thereof, filed December 19, 2011.
V. International Patent Application Publication No. WO 2009/055720, Hossack, et al., System for Treatment and Imaging Using Ultrasonic Energy and Microbubbles and Related Method Thereof, April 30, 2009.
W. U.S. Patent No. 8,622,911, Hossack, et al., System for Treatment and Imaging Using Ultrasonic Energy and Microbubbles and Related Method Thereof, issued January 7, 2014.
X. International Patent Application Publication No. WO 2011/011539, Hossack, et al., Systems and Methods for Ultrasound Imaging and Insonation of Microbubbles, January 27, 2011.
Y. U.S. Patent No. 9,237,898, Hossack, et al., Systems and Methods for Ultrasound Imaging and Insonation of Microbubbles, issued January 19, 2016.
Z. U.S. Patent Application Publication No. US 2014/0142468, Hossack, et al., System for Treatment and Imaging Using Ultrasonic Energy and Microbubbles and Related Method Thereof, May 22, 2014.
AA. U.S. Patent Application Publication No. US 2015/0272601 A1, Hossack, et al., Method and Apparatus for Accelerated Disintegration of Blood Clot, October 1, 2015.
BB. U.S. Patent Application Serial No. 14/964,454, Hossack, et al., Method and Apparatus for Accelerated Disintegration of Blood Clot, filed December 9, 2015.
CC. US. Patent Application Publication No. US 2010/0063399, Walker, et al., Front End Circuitry for Imaging Systems and Methods of Use, March 11, 2010.
DD. International Patent Application Publication No. WO 2010/021709, Walker, et al., Front End Circuitry for Imaging Systems and Methods of Use, February 25, 2010.
EE. U.S. Patent Application Publication No. US 2011/0137175, Hossack, et al., Tracked Ultrasound Vessel Imaging, June 9, 2011.
FF. U.S. Patent No. 9,002,080, Mauldin, et al., Singular Value Filter for Imaging or Detection, issued April 7, 2015.
GG. International Patent Application Publication No. WO 2012/148985, Mauldin, Jr., et al., "Bone Surface Image Reconstruction Using Ultrasound", November 1, 2012.
HH. U.S. Patent Application Publication No. US 2014/0046186, Mauldin, Jr., et al., "Bone Surface Image Reconstruction Using Ultrasound", February 13, 2014.
II. International Patent Application Publication No. WO 2013/188625, Mauldin, Jr., et al., "Ultrasound Imaging of Specular-Reflecting Target", December 19, 2013.
JJ. U.S. Patent Application Publication No. US 2015/0133788, Mauldin, Jr., et al., "Ultrasound Imaging of Specular-Reflecting Target", May 14, 2015.
KK. U.S. Patent Application Publication No. US 2015/0150534, Mauldin, Jr., et al.," System and Method for Binding Dynamics of Targeted Microbubbles", June 4, 2015.
LL. International Patent Application No. PCTUS15/47374, Dixon, et al., "Method and Apparatus for Accelerated Disintegration of Blood Clot", filed August 28, 2015.
MM. International Patent Application No. PCT/US2015/064543, Hu, et al., "Multispectral Photoacoustic Microscopy Based on an Optical-acoustic Objective and Related Method Thereof", filed December 8, 2015.

## Claims

1. A system (700) for effecting ultrasound-based protection from ischemia/reperfusion injury (IRI) and/or mitigating in a subject an inflammatory condition, disease or disorder, said system comprising:
an ultrasound imaging device for imaging a target tissue region;
a transducer (724) for performing a volumetric sweep through the target volume systematically for a selected total time duration and for applying ultrasonic energy to a volume region of interest (ROI) (13), wherein applying the ultrasonic energy comprises emitting a sequence of ultrasonic pulses from the transducer (724), the sequence of ultrasound pulses having predetermined frequency, mechanical index, pulse length(s), and pulse spacing(s), while performing a volumetric sweep through the ROI (13) systematically;
a storage comprising an image library database wherein said image library database provides an image comparison/similarity algorithm that is used to automatically identify the spleen in image frames from the imaging device and thereby identify an optimal ROI that encompasses it; a processor (732) for identifying the optimal ROI (13) by highlighting a region on one of the image frames as the transducer (724) is swept through a volume; and
a controller (722) for selecting a therapeutic ultrasound dose of the transducer comprising a predetermined frequency, mechanical index, pulse length, and pulse spacing,
wherein the imaging device and the transducer (724) are the same component.

2. The system (700) of claim 1, wherein said transducer (724) is placed in a mechanical translation stage to automatically effect the sweep, and/or
wherein said transducer is a sector transducer array, optionally wherein it steers to +/- 45 degrees, or
wherein said transducer is a curved linear transducer array.

3. The system (700) of claim 1 or claim 2, wherein said transducer (724) comprises a 2D array that can sweep through an entire target volume without physical translation.

4. The system (700) of any one of claims 1-3,wherein said selected total time duration ranges from about 3 minutes to about 15 minutes, optionally wherein said total time duration is about 10 minutes.

5. The system (700) of any one of claims 1-4, wherein the ultrasonic energy has a frequency of between about 1 and about 10 megahertz (MHz); and/or
wherein the ultrasonic energy is applied at a mechanical index ranging from about 0.5 to about 1.9.

6. The system (700) of any one of claims 1-5, wherein the sequence of ultrasound pulses comprises burst pulses having a frequency of about 1.7 MHz and imaging pulses having a frequency of about 3 MHz.

7. The system (700) of any one of claims 1-6, wherein the sequence of ultrasound pulses comprises a series of burst pulse sequences, optionally wherein the duration of each burst pulse sequence is between about 3 and about 5 seconds and the time interval between burst pulse sequences is about 1 second; further optionally ; optionally
wherein each burst pulse sequence comprises a series of pulse sub-sequences, wherein each pulse sub-sequence comprises a series of pulse repetitions at a single lateral location.

8. The system (700) of any one of claims 1-6, wherein the ultrasonic energy "on" time, relative to the time between successive pulses, lies in the range of about 1% to about 10%.

9. The system (700) of any one of claims 1-8, wherein said system measures instantaneous sweep velocity and provides and audible or visual cue as to "too fast" or "too slow".

10. A non-transitory computer readable medium having stored thereon executable instructions (424) that when executed by a processor (402) of a controller (722) of the the system (700) of claim 1 control the system (700) to perform steps comprising:
imaging spleen tissue ultrasonically within a subject; automatically identifying the spleen in images from the imaging and thereby identifying an optimal ROI that encompasses it by using an image comparison/similarity algorithm provided by an image library database comprised by storage of the system;
applying therapeutic ultrasonic energy to the ROI (13), wherein applying the ultrasonic energy comprises emitting a sequence of ultrasonic pulses from an ultrasound transducer (724) of the system (700), the sequence of ultrasound pulses having predetermined frequency, mechanical index, pulse length, and pulse spacing, while performing a volumetric sweep through the ROI (13) systematically for a selected total time duration of between about 5 minutes and about 15 minutes, optionally wherein the total time duration is about 10 minutes.

11. The non-transitory computer readable medium of claim 10, wherein the ultrasonic energy has a frequency of between about 1 and about 10 megahertz (MHz); and/or
wherein the sequence of ultrasound pulses comprises burst pulses having a frequency of about 1.7 MHz and imaging pulses having a frequency of about 3 MHz; and/or
wherein the ultrasonic energy is applied at a mechanical index ranging from about 0.5 to about 1.9.

12. The non-transitory computer readable medium of claim 10 or claim 11, wherein the sequence of ultrasound pulses comprises a series of burst pulse sequences, optionally wherein the duration of each burst pulse sequence is between about 3 and about 5 seconds and the time interval between burst pulse sequences is about 1 second; and/or
wherein the ultrasonic energy "on" time, relative to the time between successive pulses, lies in the range of about 1% to about 10%; optionally
wherein each burst pulse sequence comprises a series of pulse sub-sequences, further optionally wherein each pulse sub-sequence comprises a series of pulse repetitions at a single lateral location.

13. A computer program comprising executable instructions (424) that when executed by a processor (402) of a controller (722) of the the system (700) of claim 1 control the system (700) to perform steps comprising:
imaging spleen tissue ultrasonically within a subject; automatically identifying the spleen in images from the imaging and thereby identifying an optimal ROI that encompasses it by using an image comparison/similarity algorithm provided by an image library database comprised by storage of the system;
applying therapeutic ultrasonic energy to the ROI (13), wherein applying the ultrasonic energy comprises emitting a sequence of ultrasonic pulses from an ultrasound transducer (724) of the system (700), the sequence of ultrasound pulses having predetermined frequency, mechanical index, pulse length, and pulse spacing, while performing a volumetric sweep through the ROI (13) systematically for a selected total time duration of between about 5 minutes and about 15 minutes, optionally wherein the total time duration is about 10 minutes.

14. The computer program of claim 13, wherein the ultrasonic energy has a frequency of between about 1 and about 10 megahertz (MHz); and/or
wherein the sequence of ultrasound pulses comprises burst pulses having a frequency of about 1.7 MHz and imaging pulses having a frequency of about 3 MHz; and/or
wherein the ultrasonic energy is applied at a mechanical index ranging from about 0.5 to about 1.9.

15. The computer program of claim 13 or 14, wherein the sequence of ultrasound pulses comprises a series of burst pulse sequences, optionally wherein the duration of each burst pulse sequence is between about 3 and about 5 seconds and the time interval between burst pulse sequences is about 1 second; and/or
wherein the ultrasonic energy "on" time, relative to the time between successive pulses, lies in the range of about 1% to about 10%; optionally
wherein each burst pulse sequence comprises a series of pulse sub-sequences, further optionally wherein each pulse sub-sequence comprises a series of pulse repetitions at a single lateral location.

## Patentansprüche

1. System (700) zum Herbeiführen eines ultraschallbasierten Schutzes vor einer Ischämie-Reperfusionsverletzung (*Ischemia*/*Reperfusion Injury*, IRI) und/oder zur Linderung eines entzündlichen Zustands, einer entzündlichen Krankheit oder einer entzündlichen Störung bei einer Person, wobei das System umfasst:
eine Ultraschallbildgabevorrichtung zum Abbilden einer Zielgeweberegion;
einen Messwandler (724) zum Durchführen einer systematischen volumetrischen Bestreichung des Zielvolumens über eine ausgewählte Gesamtzeitdauer und zum Anlegen von Ultraschallenergie an eine interessierende Volumenregion (*Region Of Interest*, ROI) (13),
wobei das Anlegen der Ultraschallenergie das Aussenden einer Sequenz von Ultraschallimpulsen von dem Messwandler (724) umfasst, wobei die Sequenz von Ultraschallimpulsen eine zuvor festgelegte Frequenz, einen zuvor festgelegten mechanischen Index, eine oder mehrere zuvor festgelegte Impulslängen und einen oder mehrere zuvor festgelegte Impulsabstände aufweist, während systematisch eine volumetrische Bestreichung der ROI (13) durchgeführt wird;
einen Speicher, der eine Bildbibliotheksdatenbank umfasst,
wobei die Bildbibliotheksdatenbank einen Bildvergleichs-/Ähnlichkeitsalgorithmus bereitstellt, der verwendet wird, um automatisch die Milz in Bild-Frames von der Bildgabevorrichtung zu identifizieren und dadurch eine optimale ROI zu ermitteln, die sie umfasst;
einen Prozessor (732) zum Identifizieren der optimalen ROI (13) durch Hervorheben einer Region auf einem der Bild-Frames, wenn der Messwandler (724) ein Volumen bestreicht; und
einen Controller (722) zum Auswählen einer therapeutischen Ultraschalldosis des Messwandlers, die eine zuvor festgelegte Frequenz, einen zuvor festgelegten mechanischen Index, eine zuvor festgelegte Impulslänge und einen zuvor festgelegten Impulsabstand umfasst,
wobei die Bildgabevorrichtung und der Messwandler (724) dieselbe Komponente sind.

2. System (700) nach Anspruch 1,
wobei der Messwandler (724) in einem mechanischen Translationstisch platziert wird, um das Bestreichen automatisch durchzuführen, und/oder
wobei der Messwandler eine Sektor-Messwandleranordnung ist,
wobei er optional auf ±45 Grad steuert, oder
wobei der Messwandler eine gekrümmte lineare Messwandleranordnung ist.

3. System (700) nach Anspruch 1 oder Anspruch 2,
wobei der Messwandler (724) eine 2D-Anordnung umfasst, die ein gesamtes Zielvolumen ohne physische Translation bestreichen kann.

4. System (700) nach einem der Ansprüche 1-3,
wobei die ausgewählte Gesamtzeitdauer im Bereich von etwa 3 Minuten bis etwa 15 Minuten liegt,
wobei optional die Gesamtzeitdauer etwa 10 Minuten beträgt.

5. System (700) nach einem der Ansprüche 1-4,
wobei die Ultraschallenergie eine Frequenz zwischen etwa 1 und etwa 10 Megahertz (MHz) aufweist; und/oder
wobei die Ultraschallenergie bei einem mechanischen Index im Bereich von etwa 0,5 bis etwa 1,9 angelegt wird.

6. System (700) nach einem der Ansprüche 1-5,
wobei die Sequenz von Ultraschallimpulsen Burst-Impulse mit einer Frequenz von etwa 1,7 MHz und Bildgabeimpulse mit einer Frequenz von etwa 3 MHz umfasst.

7. System (700) nach einem der Ansprüche 1-6,
wobei die Sequenz von Ultraschallimpulsen eine Reihe von Burst-Impulssequenzen umfasst,
wobei optional die Dauer jeder Burst-Impulssequenz zwischen etwa 3 und etwa 5 Sekunden beträgt und das Zeitintervall zwischen Burst-Impulssequenzen etwa 1 Sekunde beträgt;
wobei des Weiteren optional jede Burst-Impulssequenz eine Reihe von Impuls-Teilsequenzen umfasst,
wobei jede Impuls-Teilsequenz eine Reihe von Impulswiederholungen an einer einzelnen lateralen Stelle umfasst.

8. System (700) nach einem der Ansprüche 1-6,
wobei die Ein-Zeit der Ultraschallenergie, relativ zu der Zeit zwischen aufeinanderfolgenden Impulsen, im Bereich von etwa 1 % bis etwa 10 % liegt.

9. System (700) nach einem der Ansprüche 1-8,
wobei das System die augenblickliche Bestreichungsgeschwindigkeit misst und einen akustischen oder visuellen Hinweis mit der Bedeutung "zu schnell" oder "zu langsam" ausgibt.

10. Nicht-transitorisches computerlesbares Medium, auf dem ausführbare Instruktionen (424) gespeichert sind, die, wenn sie durch einen Prozessor (402) eines Controllers (722) des Systems (700) von Anspruch 1 ausgeführt werden, das System (700) veranlassen, Schritte durchzuführen, umfassend:
Ultraschallbildgeben des Milzgewebes bei einer Person;
automatisches Identifizieren der Milz in Bildern von der Bildgabe und dadurch Identifizieren einer optimalen ROI, die sie umfasst, unter Verwendung eines Bildvergleichs-/Ähnlichkeitsalgorithmus, der durch eine Bildbibliotheksdatenbank bereitgestellt wird, die sich im Speicher des Systems befindet;
Anlegen therapeutischer Ultraschallenergie an die ROI (13),
wobei das Anlegen der Ultraschallenergie das Aussenden einer Sequenz von Ultraschallimpulsen von einem Ultraschallmesswandler (724) des Systems (700) umfasst, wobei die Sequenz von Ultraschallimpulsen eine zuvor festgelegte Frequenz, einen zuvor festgelegten mechanischen Index, eine zuvor festgelegte Impulslänge und einen zuvor festgelegten Impulsabstand aufweist, während eine volumetrische Bestreichung der ROI (13) systematisch über eine ausgewählte Gesamtzeitdauer zwischen etwa 5 Minuten und etwa 15 Minuten durchgeführt wird,
wobei optional die Gesamtzeitdauer etwa 10 Minuten beträgt.

11. Nicht-transitorisches computerlesbares Medium nach Anspruch 10,
wobei die Ultraschallenergie eine Frequenz zwischen etwa 1 und etwa 10 Megahertz (MHz) aufweist; und/oder
wobei die Sequenz von Ultraschallimpulsen Burst-Impulse mit einer Frequenz von etwa 1,7 MHz und Bildgabeimpulse mit einer Frequenz von etwa 3 MHz umfasst; und/oder
wobei die Ultraschallenergie bei einem mechanischen Index im Bereich von etwa 0,5 bis etwa 1,9 angelegt wird.

12. Nicht-transitorisches computerlesbares Medium nach Anspruch 10 oder Anspruch 11,
wobei die Sequenz von Ultraschallimpulsen eine Reihe von Burst-Impulssequenzen umfasst,
wobei optional die Dauer jeder Burst-Impulssequenz zwischen etwa 3 und etwa 5 Sekunden beträgt und das Zeitintervall zwischen Burst-Impulssequenzen etwa 1 Sekunde beträgt; und/oder
wobei die Ein-Zeit der Ultraschallenergie, relativ zu der Zeit zwischen aufeinanderfolgenden Impulsen, im Bereich von etwa 1 % bis etwa 10 % liegt;
wobei optional jede Burst-Impulssequenz eine Reihe von Impuls-Teilsequenzen umfasst,
wobei des Weiteren optional jede Impuls-Teilsequenz eine Reihe von Impulswiederholungen an einer einzelnen lateralen Stelle umfasst.

13. Computerprogramm, umfassend ausführbare Instruktionen (424), die, wenn sie durch einen Prozessor (402) eines Controllers (722) des Systems (700) nach Anspruch 1 ausgeführt werden, das System (700) veranlassen, Schritte durchzuführen, umfassend:
Ultraschallbildgeben des Milzgewebes bei einer Person;
automatisches Identifizieren der Milz in Bildern von der Bildgabe und dadurch Identifizieren einer optimalen ROI, die sie umfasst, unter Verwendung eines Bildvergleichs-/Ähnlichkeitsalgorithmus, der durch eine Bildbibliotheksdatenbank bereitgestellt wird, die sich im Speicher des Systems befindet;
Anlegen therapeutischer Ultraschallenergie an die ROI (13),
wobei das Anlegen der Ultraschallenergie das Aussenden einer Sequenz von Ultraschallimpulsen von einem Ultraschallmesswandler (724) des Systems (700) umfasst, wobei die Sequenz von Ultraschallimpulsen eine zuvor festgelegte Frequenz, einen zuvor festgelegten mechanischen Index, eine zuvor festgelegte Impulslänge und einen zuvor festgelegten Impulsabstand aufweist, während eine volumetrische Bestreichung der ROI (13) systematisch über eine ausgewählte Gesamtzeitdauer zwischen etwa 5 Minuten und etwa 15 Minuten durchgeführt wird,
wobei optional die Gesamtzeitdauer etwa 10 Minuten beträgt.

14. Computerprogramm nach Anspruch 13,
wobei die Ultraschallenergie eine Frequenz zwischen etwa 1 und etwa 10 Megahertz (MHz) aufweist; und/oder
wobei die Sequenz von Ultraschallimpulsen Burst-Impulse mit einer Frequenz von etwa 1,7 MHz und Bildgabeimpulse mit einer Frequenz von etwa 3 MHz umfasst; und/oder
wobei die Ultraschallenergie bei einem mechanischen Index im Bereich von etwa 0,5 bis etwa 1,9 angelegt wird.

15. Computerprogramm nach Anspruch 13 oder 14,
wobei die Sequenz von Ultraschallimpulsen eine Reihe von Burst-Impulssequenzen umfasst,
wobei optional die Dauer jeder Burst-Impulssequenz zwischen etwa 3 und etwa 5 Sekunden beträgt und das Zeitintervall zwischen Burst-Impulssequenzen etwa 1 Sekunde beträgt; und/oder
wobei die Ein-Zeit der Ultraschallenergie, relativ zu der Zeit zwischen aufeinanderfolgenden Impulsen, im Bereich von etwa 1 % bis etwa 10 % liegt;
wobei optional jede Burst-Impulssequenz eine Reihe von Impuls-Teilsequenzen umfasst,
wobei des Weiteren optional jede Impuls-Teilsequenz eine Reihe von Impulswiederholungen an einer einzelnen lateralen Stelle umfasst.

## Revendications

1. Système (700) permettant de mettre en œuvre une protection à base d'ultrasons contre une lésion d'ischémie-reperfusion (IRI, *ischemia*/*reperfusion injury*) et/ou d'atténuer chez un sujet un état, une maladie ou un trouble inflammatoire, ledit système comprenant :
un dispositif d'imagerie ultrasonore permettant d'imager une région tissulaire cible ;
un transducteur (724) permettant de réaliser de façon systématique un balayage volumétrique à travers le volume cible pendant une durée totale choisie et d'appliquer une énergie ultrasonore à une région de volume d'intérêt (ROI, *region of interest*) (13), dans lequel l'application de l'énergie ultrasonore comprend l'émission d'une séquence d'impulsions ultrasonores à partir du transducteur (724), la séquence d'impulsions ultrasonores ayant une fréquence, un indice mécanique, une ou des longueur(s) d'impulsions, un ou des espacement(s) entre impulsions prédéterminés, pendant la réalisation systématique d'un balayage volumétrique à travers la région ROI (13) ;
une mémoire comprenant une base de données de bibliothèque d'images, dans laquelle ladite base de données de bibliothèque d'images procure un algorithme de comparaison/similarité d'images qui est utilisé pour identifier automatiquement la rate sur des trames d'image issues du dispositif d'imagerie et identifier ainsi une région ROI optimale qui l'englobe ;
un processeur (732) permettant d'identifier la région ROI optimale (13) en mettant en évidence une région sur l'une des trames d'image à mesure que le transducteur (724) balaie un volume ; et
une unité de commande (722) permettant de sélectionner une dose thérapeutique d'ultrasons du transducteur, comprenant une fréquence, un indice mécanique, une longueur d'impulsions et un espacement entre impulsions prédéterminés,
dans lequel le dispositif d'imagerie et le transducteur (724) sont un même composant.

2. Système (700) selon la revendication 1, dans lequel ledit transducteur (724) est placé sur une platine de translation mécanique pour effectuer le balayage automatiquement, et/ou
dans lequel ledit transducteur est un réseau de transducteurs sectoriels, éventuellement dans lequel il peut être orienté à +/- 45 degrés, ou
dans lequel ledit transducteur est un réseau de transducteurs linéaires courbe.

3. Système (700) selon la revendication 1 ou la revendication 2, dans lequel ledit transducteur (724) comprend un réseau en 2D qui peut balayer un volume cible entier sans translation physique.

4. Système (700) selon l'une quelconque des revendications 1 à 3, dans lequel ladite durée totale choisie est comprise entre environ 3 minutes et environ 15 minutes, éventuellement dans lequel ladite durée totale est d'environ 10 minutes.

5. Système (700) selon l'une quelconque des revendications 1 à 4, dans lequel l'énergie ultrasonore a une fréquence comprise entre environ 1 et environ 10 mégahertz (MHz) ; et/ou
dans lequel l'énergie ultrasonore est appliquée à un indice mécanique compris entre environ 0,5 et environ 1,9.

6. Système (700) selon l'une quelconque des revendications 1 à 5, dans lequel la séquence d'impulsions ultrasonores comprend des impulsions en rafale ayant une fréquence d'environ 1,7 MHz et des impulsions d'imagerie ayant une fréquence d'environ 3 MHz.

7. Système (700) selon l'une quelconque des revendications 1 à 6, dans lequel la séquence d'impulsions ultrasonores comprend une série de séquences d'impulsions en rafales, éventuellement dans lequel la durée de chaque séquence d'impulsions en rafale est comprise entre environ 3 et environ 5 secondes et l'intervalle de temps entre des séquences d'impulsions en rafales est d'environ 1 seconde ; éventuellement en outre :
dans lequel chaque séquence d'impulsions en rafale comprend une série de sous-séquences d'impulsions, dans lequel chaque sous-séquence d'impulsions comprend une série de répétitions d'impulsions à un seul emplacement latéral.

8. Système (700) selon l'une quelconque des revendications 1 à 6, dans lequel le temps « haut » de l'énergie ultrasonore par rapport au temps entre des impulsions successives se situe dans la plage d'environ 1 % à environ 10 %.

9. Système (700) selon l'une quelconque des revendications 1 à 8, dans lequel ledit système mesure la vitesse instantanée de balayage et fournit un signal sonore ou visuel indiquant « trop rapide » ou « trop lent ».

10. Support non transitoire lisible par ordinateur, sur lequel sont stockées des instructions exécutables (424) qui, lorsqu'elles sont exécutées par un processeur (402) d'unité de commande (722) du système (700) selon la revendication 1, font effectuer au système (700) les étapes comprenant :
imager du tissu splénique par ultrasons chez un sujet ;
identifier automatiquement la rate sur des images issues de l'imagerie et identifier ainsi une région ROI optimale qui l'englobe, en utilisant un algorithme de comparaison/similarité d'images procuré par une base de données de bibliothèque d'images comprise dans la mémoire du système ;
appliquer une énergie ultrasonore thérapeutique à la région ROI (13), dans lequel l'application de l'énergie ultrasonore comprend l'émission d'une séquence d'impulsions ultrasonores à partir d'un transducteur à ultrasons (724) du système (700), la séquence d'impulsions ultrasonores ayant une fréquence, un indice mécanique, une longueur d'impulsions et un espacement entre impulsions prédéterminés, tout en réalisant de façon systématique un balayage volumétrique à travers la région ROI (13) pendant une durée totale choisie comprise entre environ 5 minutes et environ 15 minutes, éventuellement dans lequel la durée totale est d'environ 10 minutes.

11. Support non transitoire lisible par ordinateur, selon la revendication 10, dans lequel l'énergie ultrasonore a une fréquence comprise entre environ 1 et environ 10 mégahertz (MHz) ; et/ou
dans lequel la séquence d'impulsions ultrasonores comprend des impulsions en rafale ayant une fréquence d'environ 1,7 MHz et des impulsions d'imagerie ayant une fréquence d'environ 3 MHz ; et/ou
dans lequel l'énergie ultrasonore est appliquée à un indice mécanique compris entre environ 0,5 et environ 1,9.

12. Support non transitoire lisible par ordinateur, selon la revendication 10 ou la revendication 11, dans lequel la séquence d'impulsions ultrasonores comprend une série de séquences d'impulsions en rafales, éventuellement dans lequel la durée de chaque séquence d'impulsions en rafale est comprise entre environ 3 et environ 5 secondes et l'intervalle de temps entre des séquences d'impulsions en rafales est d'environ 1 seconde ; et/ou
dans lequel le temps « haut » de l'énergie ultrasonore par rapport au temps entre des impulsions successives se situe dans la plage d'environ 1 % à environ 10 % ; éventuellement dans lequel chaque séquence d'impulsions en rafale comprend une série de sous-séquences d'impulsions, éventuellement en outre, dans lequel chaque sous-séquence d'impulsions comprend une série de répétitions d'impulsions à un seul emplacement latéral.

13. Programme informatique comprenant des instructions exécutables (424) qui, lorsqu'elles sont exécutées par un processeur (402) d'unité de commande (722) du système (700) selon la revendication 1, font effectuer au système (700) les étapes comprenant :
imager du tissu splénique par ultrasons chez un sujet ;
identifier automatiquement la rate sur des images issues de l'imagerie et identifier ainsi une région ROI optimale qui l'englobe, en utilisant un algorithme de comparaison/similarité d'images procuré par une base de données de bibliothèque d'images comprise dans la mémoire du système ;
appliquer une énergie ultrasonore thérapeutique à la région ROI (13), dans lequel l'application de l'énergie ultrasonore comprend l'émission d'une séquence d'impulsions ultrasonores à partir d'un transducteur à ultrasons (724) du système (700), la séquence d'impulsions ultrasonores ayant une fréquence, un indice mécanique, une longueur d'impulsions et un espacement entre impulsions prédéterminés, tout en réalisant de façon systématique un balayage volumétrique à travers la région ROI (13) pendant une durée totale choisie comprise entre environ 5 minutes et environ 15 minutes, éventuellement dans lequel la durée totale est d'environ 10 minutes.

14. Programme informatique selon la revendication 13, dans lequel l'énergie ultrasonore a une fréquence comprise entre environ 1 et environ 10 mégahertz (MHz) ; et/ou
dans lequel la séquence d'impulsions ultrasonores comprend des impulsions en rafale ayant une fréquence d'environ 1,7 MHz et des impulsions d'imagerie ayant une fréquence d'environ 3 MHz ; et/ou
dans lequel l'énergie ultrasonore est appliquée à un indice mécanique compris entre environ 0,5 et environ 1,9.

15. Programme informatique selon la revendication 13 ou 14, dans lequel la séquence d'impulsions ultrasonores comprend une série de séquences d'impulsions en rafales, éventuellement dans lequel la durée de chaque séquence d'impulsions en rafale est comprise entre environ 3 et environ 5 secondes et l'intervalle de temps entre des séquences d'impulsions en rafales est d'environ 1 seconde ; et/ou
dans lequel le temps « haut » de l'énergie ultrasonore par rapport au temps entre des impulsions successives se situe dans la plage d'environ 1 % à environ 10 % ; éventuellement dans lequel chaque séquence d'impulsions en rafale comprend une série de sous-séquences d'impulsions, éventuellement en outre, dans lequel chaque sous-séquence d'impulsions comprend une série de répétitions d'impulsions à un seul emplacement latéral.
